# EUROPEAN PATENT APPLICATION

(11) **EP 4 650 432 A1**
(43) Date of publication of application: **19.11.2025**
(21) Application number: 24741597.9
(22) Date of filing: 12.01.2024
(51) Int. Cl.: C12M 1/42, C12M 1/00

(54) **MOLECULE INTRODUCTION DEVICE**

(30) Priority: 12.01.2023 JP 2023003018; 12.01.2023 JP 2023003264
(71) Applicant: Sekisui Chemical Co., Ltd., Osaka-shi, Osaka 530-8565 (JP)
(72) Inventor: OSHITA Takaya, Tsukuba-shi, Ibaraki 300-4292 (JP); INOUE Hitoshi, Tsukuba-shi, Ibaraki 300-4292 (JP); KAMAGA Sei, Tsukuba-shi, Ibaraki 300-4292 (JP)
(74) Representative: Ter Meer Steinmeister & Partner
(86) International application number: PCT/JP2024/000667
(87) International publication number: WO 2024/150825

(57) **Abstract**

A molecule introduction device (1) including a first electrode (10); a second electrode (20) that is spaced apart from the first electrode (10) and positioned in a direction in which a distal end of the first electrode (10) faces; an irradiation field that is positioned between the first electrode (10) and the second electrode (20), and positioned to be spaced apart from the distal end of the first electrode (10); and a power supply unit (30) that applies a voltage between the first electrode (10) and the second electrode (20), in which the first electrode (10) has an irradiation body (14) at a distal end on a side of the second electrode (20), the irradiation body (14) has conductivity and has a linear portion having a linear shape in a plan view as viewed from the second electrode (20) toward the first electrode (10), and in the plan view, the linear portion has a width of 1 µm to 1,000 µm and a length of more than 3.14 mm.

## Description

### TECHNICAL FIELD

The present invention relates to a molecule introduction device.

Priority is claimed on Japanese Patent Application Nos. 2023-003018 and 2023-003264, filed on January 12, 2023, the contents of which are incorporated herein by reference.

### BACKGROUND ART

In genetic engineering and pharmaceutical drug development, molecules are introduced into target cells or target tissues (which may hereinafter be referred to as "target cells and the like"), and a function, a physiological activity, and the like of the molecules in the target cells and the like are tested. Examples of the molecule include a polynucleotide, a signal transduction protein, a transcriptional regulatory factor, a physiologically active low-molecular-weight substance, and a drug candidate.

Examples of a method of introducing the molecules include an electroporation method, a gene gun method, a ribosome method, a cell fusion method, and a virus vector method. These methods for introducing molecules have not yet achieved a satisfactory efficiency in introducing molecules into target cells and the like.

In order to solve such a problem, Patent Document 1 proposes a molecule introduction device having a capillary electrode, a counter electrode provided on a side opposite to the capillary electrode, and a power supply connected to the capillary electrode and the counter electrode.

### Citation List

### Patent Document

Patent Document 1: Japanese Patent No. 6189019

### SUMMARY OF INVENTION

### Technical Problem

However, the molecule introduction device is required to further increase an efficiency of introducing molecules into target cells and the like.

Therefore, an object of the present invention is to provide a molecule introduction device capable of further increasing an efficiency of introducing molecules.

### Solution to Problem

The present invention has the following embodiments.
<1> A molecule introduction device including:
   a first electrode;
   a second electrode that is spaced apart from the first electrode and positioned in a direction in which a distal end of the first electrode faces;
   an irradiation field that is positioned between the first electrode and the second electrode, and positioned to be spaced apart from the distal end of the first electrode; and
   a power supply unit configured to apply a voltage between the first electrode and the second electrode,
   in which the first electrode has an irradiation body at a distal end on a second electrode side,
   the irradiation body has conductivity and has a linear portion having a linear shape in a plan view as viewed from the second electrode toward the first electrode, and
   in the plan view, the linear portion has a width of 1 µm to 1,000 µm and a length of more than 3.14 mm.
<2> The molecule introduction device according to <1>,
   in which the irradiation body has, as the linear portion, one or more linear wall portions in the plan view, and
   the wall portion has a cylindrical or polygonal tubular shape with its tube axis aligned with a direction from the first electrode toward the second electrode.
<3> The molecule introduction device according to <2>,
   in which an outer diameter of the wall portion is more than 1 mm.
<4> The molecule introduction device according to <2> or <3>,
   in which the irradiation body has two or more of the wall portions, and
   the two or more wall portions form a multiple-wall structure and are spaced apart from each other.
<5> The molecule introduction device according to any one of <2> to <4>,
   in which the wall portion is a wound body with its tube axis aligned with the direction from the first electrode toward the second electrode, and
   adjacent wall portions are spaced apart from each other.
<6> The molecule introduction device according to any one of <2> to <5>,
   in which the irradiation body has an insulating core portion, and
   at least one of the wall portions is positioned on an outer surface of the insulating core portion.
<7> The molecule introduction device according to <1>,
   in which the irradiation body has, as the linear portion, one or more first conductive wires extending in one surface direction in the plan view and one or more second conductive wires intersecting the first conductive wires.
<8> The molecule introduction device according to <7>,
   in which the first electrode has an electrode body,
   the electrode body has a dielectric that extends in a direction from the first electrode toward the second electrode, and a conductive coating film configured to cover a part or an entirety of an outer surface of the dielectric,
   the conductive coating film is connected to the power supply unit, and
   the first conductive wire and the second conductive wire are electrically connected to the conductive coating film.
<9> The molecule introduction device according to <7> or <8>,
   in which a width of the first conductive wire is 1 to 1,000 µm, and
   a width of the second conductive wire is 1 to 1,000 µm.
<10> The molecule introduction device according to any one of <7> to <9>,
   in which two or more of the first conductive wires are provided, with a separation distance between the first conductive wires of 0.1 to 10 mm, and
   two or more of the second conductive wires are provided, with a separation distance between the second conductive wires of 0.1 to 10 mm.
<11> The molecule introduction device according to any one of <7> to <10>,
   in which in the plan view, the first conductive wire and the second conductive wire form at least one polygon.
<12> The molecule introduction device according to <7> to <10>,
   in which in the plan view, the first conductive wire and the second conductive wire form a plurality of polygons, and
   the polygon is a triangle, a quadrangle, or a hexagon.
<13> The molecule introduction device according to any one of <1> to <6>,
   in which in the plan view, a width of the linear portion is 1 µm to 1,000 µm, and
   the length of the linear portion per cm² is 5 mm to 320 mm.
<14> The molecule introduction device according to any one of <7> to <12>,
   in which in the plan view, the width of the linear portion is 1 µm to 1,000 µm, and
   a total length of the first conductive wire and the second conductive wire (or a total length of the linear portions) per cm² is 20 mm to 1,000 mm.

### Advantageous Effects of Invention

According to the molecule introduction device of the present invention, the efficiency of introducing molecules can be further increased.

### BRIEF DESCRIPTION OF DRAWINGS

[FIG. 1] A cross-sectional view of a molecule introduction device according to a first embodiment of the present invention.
[FIG. 2] An end view of an irradiation body 14 in FIG. 1.
[FIG. 3] An end view of another example of the irradiation body.
[FIG. 4] An end view of another example of the irradiation body.
[FIG. 5] A longitudinal sectional view of another example of the irradiation body.
[FIG. 6] A photograph showing the results of Example 1.
[FIG. 7] A photograph showing the results of Example 2.
[FIG. 8] A cross-sectional view of a molecule introduction device according to a second embodiment of the present invention.
[FIG. 9] An end view of an irradiation body 14 in FIG. 8.
[FIG. 10] A photograph showing the results of Example 3.
[FIG. 11] A photograph showing the results of Example 4.

### DESCRIPTION OF EMBODIMENTS

In the present specification and claims, "to" showing a numerical range means that numerical values described before and after the numerical range are included as the lower limit value and the upper limit value.

### (Molecule Introduction Device)

The molecule introduction device of the present invention includes a first electrode, a second electrode, and an irradiation field positioned between the first electrode and the second electrode. In addition, the first electrode has an irradiation body at a distal end on the second electrode side, the irradiation body has conductivity and has a linear portion having a linear shape in a plan view as viewed from the second electrode toward the first electrode, and in the plan view, the linear portion has a width of 1 µm to 1,000 µm and a length of more than 3.14 mm.

The molecule introduction device introduces molecules into target cells, target tissues, or the like (collectively referred to as "target cells and the like"). The molecule introduction device generates plasma from the first electrode, irradiates an irradiation target where target cells and the like coexist with molecules, and introduces the molecules into the target cells and the like.

Hereininbelow, various embodiments of the molecule introduction device of the present invention are described with reference to the accompanying drawings.

### - First Embodiment -

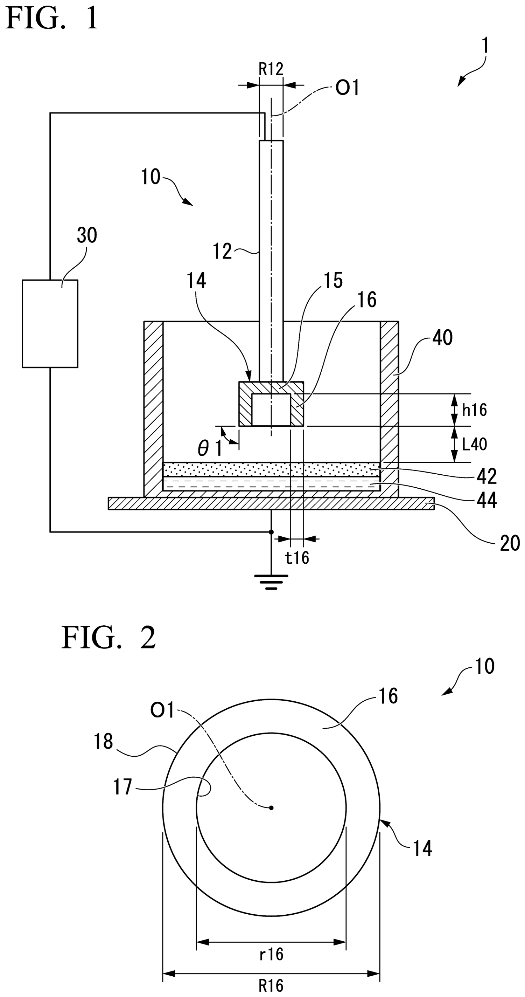

FIG. 1 is a cross-sectional view of a molecule introduction device according to a first embodiment of the present invention.

A molecule introduction device 1 in FIG. 1 has a first electrode 10, a second electrode 20, a power supply unit 30, and a container 40.

The second electrode 20 is spaced apart from the first electrode 10 and positioned below a lower end (distal end) of the first electrode 10. That is, the second electrode 20 is positioned beyond the distal end of the first electrode 10, and the first electrode 10 and the second electrode 20 face each other.

The power supply unit 30 is connected to the first electrode 10 and the second electrode 20.

The container 40 is positioned between the first electrode 10 and the second electrode 20. The container 40 is disposed to face the second electrode 20 and spaced apart from the first electrode 10.

The first electrode 10 has an electrode body 12 and an irradiation body 14 provided at a distal end of the electrode body 12. Furthermore, in the present invention, the first electrode 10 may be composed of only the irradiation body 14.

In the present embodiment, the first electrode 10 is a high-voltage electrode.

The electrode body 12 is a rod-like body that extends in one direction.

The electrode body 12 may have a hollow structure such as a cylindrical shape and a polygonal tubular shape, or may also have a solid structure such as a columnar shape and a polygonal prismatic shape. In a case where the electrode body 12 has the solid structure among those, the durability improves and the production is easy.

Examples of the material of the electrode body 12 include metals such as stainless steel, copper, and tungsten, and carbon.

A thickness (outer diameter) R12 of the electrode body 12 is, for example, 1 to 50 mm. In a case where the electrode body 12 has the polygonal cylindrical shape or the polygonal prismatic shape, the outer diameter R12 is a diameter of the circumscribed circle of the transverse cross-section of the electrode body 12.

The irradiation body 14 has a flat plate-shaped substrate portion 15 and a wall portion 16 that hangs down from a peripheral edge of the substrate portion 15. The substrate portion 15 and the wall portion 16 are connected to each other. FIG. 2 is an end surface of the irradiation body 14 as viewed from the lower end (that is, the direction of the second electrode 20) of the first electrode 10. As shown in FIG. 2, the wall portion 16 has a hollow interior and has a cylindrical shape that is open at a lower end (distal end). The wall portion 16 is a single cylinder with its tube axis O1 aligned with a direction from the first electrode 10 to the second electrode 20, and has a linear shape in the plan view as viewed from the second electrode 20 toward the first electrode 10. That is, the wall portion 16 is a linear portion of the irradiation body 14. Furthermore, the wall portion 16 is not limited to the cylindrical shape and may also have a polygonal prismatic shape.

It should be noted that in the first embodiment, the width (the thickness of the wall portion) of the linear portion in the plan view is 1 µm to 1,000 µm, preferably 10 µm to 500 µm, and more preferably 100 µm to 300 µm. Here, a thickness of the wall portion may be uniform or may be non-uniform over the entire wall portion. For example, the wall portion may have a tapered shape in which a thickness gradually decreases from a top portion to a bottom portion. In a case of the wall portion having such a tapered shape, a configuration may be adopted in which a thickness of the wall portion at the bottom portion is within the range of the width and a thickness of the wall portion at the top portion is outside the range of the width (for example, 2,000 to 3,000 µm).

Further, the length of the linear portion in the plan view is more than 3.14 mm, preferably 5 mm to 320 mm, and more preferably 10 mm to 110 mm. In a case where the width and the length are within the above ranges, the efficiency of introducing molecules can be further remarkably increased. Furthermore, in a case where a plurality of the wall portions are present, the length is a length per wall portion. In addition, in a case where a plurality of the wall portions are present, the widths of the wall portions may be different from each other, but it is preferable that the widths of all the wall portions are within the above range.

The material of the substrate portion 15 is not particularly limited as long as it is conductive, and examples thereof include metals such as stainless steel, copper, and tungsten, and carbon.

The material of the wall portion 16 is not particularly limited as long as it is conductive, and examples thereof include metals such as stainless steel, copper, and tungsten, and carbon.

The substrate portion 15 and the wall portion 16 may be an integrally molded product, or may be individually molded and joined.

In a case where the electrode body 12 has a hollow structure, the substrate portion 15 may or may not have a through-hole that allows the inside of the electrode body 12 and the inside of the wall portion 16 to communicate with each other.

The thickness (outer diameter) R16 of the wall portion 16 can be appropriately determined in consideration of the size of the container 40 and the like. The outer diameter R16 is, for example, preferably more than 1 mm, more preferably 3 to 50 mm, and still more preferably 5 to 10 mm. In a case where the outer diameter R16 is equal to or more than the lower limit value, the efficiency of introducing the molecules into the target cells and the like (which may hereinafter be simply referred to as an "introduction efficiency") can be further increased by performing the plasma irradiation over a wider range. In a case where the outer diameter R16 is equal to or less than the upper limit value, discharge can be more stably performed.

In addition, it is preferable that the outer diameter R16 is larger than the outer diameter R12. In a case where the outer diameter R16 is large, the introduction efficiency can be further increased by performing the plasma irradiation over a wider range.

In a case where the wall portion 16 has a polygonal tubular shape, the outer diameter R16 is a diameter of a circumscribed circle of a transverse cross-section of the wall portion 16.

In a case where the irradiation body 14 has two or more wall portions 16, the outer diameter R16 of the wall portion 16 is the outer diameter of the wall portion 16 positioned on the outermost side.

The inner diameter r16 of the wall portion 16 is not particularly limited and can be appropriately determined according to the outer diameter R16 and a thickness t16 of the wall portion 16.

An angle θ1 formed by the lower end surface of the wall portion 16 and the outer surface of the wall portion 16 is, for example, preferably 60° or less, more preferably 30° or less, and still more preferably 10° or less. The intersection between the lower end surface of the wall portion 16 and the outer surface of the wall portion 16 is a so-called "edge". In a case where the angle θ1 is equal to or less than the upper limit value, an electric field is concentrated on the edge, and plasma can be more easily generated between the electrodes. The lower limit value of the angle θ is, for example, 1° or more.

An angle formed by the lower end surface of the wall portion 16 and the inner surface of the wall portion 16 is the same as the angle θ1. The angle formed by the lower end surface of the wall portion 16 and the inner surface of the wall portion 16 may be the same as or different from the angle θ1.

The thickness t16 of the wall portion 16 is, for example, preferably 1 mm or less, and more preferably 0.1 mm or less. In a case where the thickness t16 is equal to or less than the upper limit value, the electric field at the end portion can be further increased. The lower limit value of the thickness t16 is, for example, 0.001 mm or more.

A height h16 of the wall portion 16 is not particularly limited, and is, for example, preferably 1 to 3 mm.

The second electrode 20 may function as a ground electrode, and examples thereof include a flat plate-shaped electrode.

Examples of a material of the second electrode 20 include metals such as stainless steel, copper, and tungsten, and carbon.

In the plan view, it is preferable that the area of the second electrode 20 is larger than the area of the first electrode 10. In a case where the area of the second electrode 20 is large, plasma can be irradiated from the first electrode 10 to the irradiation target over to a wider range.

The power supply unit 30 may apply a voltage between the first electrode 10 and the second electrode to generate plasma between both electrodes. The power supply unit 30 switches the start or the stop of the supply of electricity to the electrodes. In addition, the power supply unit 30 adjusts a voltage and a frequency applied to the electrodes. Examples of the power supply unit 30 include a circuit including an inverter that is connected to an external power supply. In addition, examples of the power supply unit 30 include a secondary battery.

In the present embodiment, the container 40 functions as an irradiation field. The irradiation field may be any place where an irradiation target is placed in a method of introducing molecules to be described below. Therefore, in a case where the container 40 is not included in the configuration of the molecule introduction device 1, the second electrode 20 on which the container 40 is placed may be used as the irradiation field.

The container 40 may be any container as long as it can accommodate the irradiation target, and examples thereof include each well of a microplate.

The inner bottom surface of the container 40 is spaced apart from the distal end of the first electrode 10 (that is, the irradiation body 14).

The material of the container 40 may be a conductive material or an insulating material. It should be noted that from the viewpoint of preventing the occurrence of local discharge due to a high voltage, the insulating material is preferable. Examples of the insulating material include a resin, ceramics, and glass. Examples of the resin include polystyrene, a polyolefin, and a polyester.

### (Method of Using Molecule Introduction Device)

An example of a method of using the molecule introduction device (that is, a method of introducing molecules) is described below.

As an example of the method of introducing the molecules, a method having a pre-culture step, a contact step, and an irradiation step can be mentioned.

In the pre-culture step, target cells and the like are cultured. As the culturing method, a culturing method known in the related art can be adopted. Examples of the culturing method include a method of culturing target cells and the like in the container 40. For example, in a case where a culture plate is used as the container 40, the inside of a well of the culture plate is coated with a scaffold material, and the target cells and the like are cultured in a culture medium in a coated well.

After the pre-culture, the culture medium is removed from the container 40 to obtain target cells and the like 44 after the pre-culture.

Examples of the target cells and the like include animal cells including human cells, animal bodies other than human bodies, cells collected from human bodies, cells in human bodies, human bodies, plant cells, and microbial cells. Furthermore, cells collected from the human bodies include cells that are not intended to be returned to the human bodies, and cells that are intended to be returned to human bodies to used for regenerative medicine or the like. In addition, the cells collected from the human bodies include cells cultured from the cells collected from the human bodies.

The culture medium used for the pre-culture is not particularly limited, and is appropriately selected according to the purpose.

The culture conditions for the pre-culture step are, for example, 30°C to 40°C, and 24 to 72 hours.

The number of target cells and the like 44 after the pre-culture is appropriately determined in consideration of the capacity of the container 40, and the like. For example, in a case where the container 40 is a 96-well culture plate, for example, the number of cells is set to 1 × 10³ to 1 × 10⁶ cells per well. In addition, for example, in a case where the container 40 is a 6-well culture plate, the number of cells is set to 1 × 10⁴ to 1 × 10⁷ per well.

The contact step is a step of allowing the target cells and the molecules to coexist and contact each other.

The molecules 42 are added to the container 40 from which the culture medium has been removed. In this case, the molecules may be in the form of an aqueous solution or a dispersion liquid (collectively referred to as "aqueous liquid"), and the aqueous liquid may be added to the container.

The molecules 42 are molecules selected for the introduction into the target cells and the like 44. Examples of the molecules 42 include a polymer compound, a physiologically active low-molecular-weight substance, and a drug candidate.

Examples of the polymer compound include a polynucleotide such as DNA and RNA, or a derivative thereof, a protein or peptide such as a signal transduction protein and a transcriptional regulatory factor, or a derivative thereof.

The molecules 42 may be used alone or in combination of two or more kinds thereof.

The irradiation step is a step of irradiating the inside of the container 40 with plasma in a state where the target cells and the like 44 and the molecules 42 coexist and come into contact with each other. By going through the irradiation step, the molecules 42 are introduced into the target cells and the like 44.

In the irradiation step, the power supply unit 30 supplies power to the first electrode 10, and a voltage is applied between the first electrode 10 and the second electrode 20.

In a case where a voltage is applied between the electrodes, electric field concentration occurs at an edge of a lower end of the wall portion 16, and plasma is generated from the electric field concentration point toward the second electrode 20. In this case, since the wall portion 16 has a cylindrical shape, the irradiation target can be irradiated with plasma over a wide range. In a case where the plasma irradiation is performed over a wide range, the molecules can be introduced into target cells and the like over a wide range of an irradiation target by performing the plasma irradiation once. Therefore, the introduction amount per unit time is increased and the introduction efficiency can be further increased.

Furthermore, the generation of plasma between the electrodes by applying a voltage between the first electrode 10 and the second electrode 20 is also referred to as plasma irradiation.

A distance L40 from the lower end of the first electrode 10 (the lower end surface of the wall portion 16) to the irradiation target (the liquid in which the molecules 42 and the target cells 44 coexist) is preferably 0.1 to 5 mm. In a case where the distance L40 is within the range, plasma can be generated more stably.

The alternating voltage applied between the first electrode 10 and the second electrode 20 is preferably 1 to 30 kVpp, and more preferably 5 to 20 kVpp. In a case where the alternating voltage is set to be equal to or more than the lower limit value, the introduction efficiency can be further increased. In a case where the alternating voltage is set to be equal to or less than the upper limit value, a damage to the target cells and the like can be further reduced. The unit "Volt peak to peak (Vpp)" representing an alternating voltage is a potential difference between a maximum value and a minimum value of an alternating voltage waveform.

The frequency of the alternating current to be applied between the first electrode 10 and the second electrode 20 is preferably 1 to 500 kHz, and more preferably 10 to 200 kHz. In a case where the frequency is set to be equal to or more than the lower limit value, the introduction efficiency can be further increased. In a case where the frequency is set to be equal to or lower than the upper limit value, the damage to the target cells and the like can be further reduced.

The irradiation time with the plasma in the irradiation step is, for example, preferably 10 nanoseconds to 1 second, and more preferably 100 nanoseconds to 0.1 seconds. In a case where the irradiation time is equal to or longer than the lower limit value, the amount of the molecules 42 introduced into the target cells and the like 44 can be further increased. In a case where the irradiation time is equal to or shorter than the upper limit value, the damage to the target cells and the like can be further reduced.

In the irradiation step, the environment in the container 40 is not particularly limited but, for example, CO₂ may be present. The CO₂ concentration is, for example, 1% to 10% by volume.

The method of introducing the molecules may have a post-culture step after the irradiation step. The post-culture step is a step of culturing the target cells and the like (which may be referred to as "introduced cells or the like") into which the molecules have been introduced.

The culture conditions in the post-culture step are the same as those in the pre-culture step, and can be appropriately determined according to kinds of the introduced cell and the like, an intended use of the introduced cells and the like, or the like. The culture conditions in the post-culture step may be the same as or different from the culture conditions in the pre-culture step.

The obtained introduced cells and the like are used for, for example, substance production, gene and cell therapy, and the like.

According to the molecule introduction device of the present embodiment, since the distal end of the first electrode has an irradiation body and the irradiation body has a cylindrical wall portion, the introduction efficiency can be increased by performing the plasma irradiation over a wide area.

### (Other Aspects of First Embodiment)

The first embodiment of the present invention is not limited to the above-described embodiment.

In the above-described embodiment, the irradiation body has a single wall portion. However, the present invention is not limited to this and the irradiation body may have multiple wall portions.

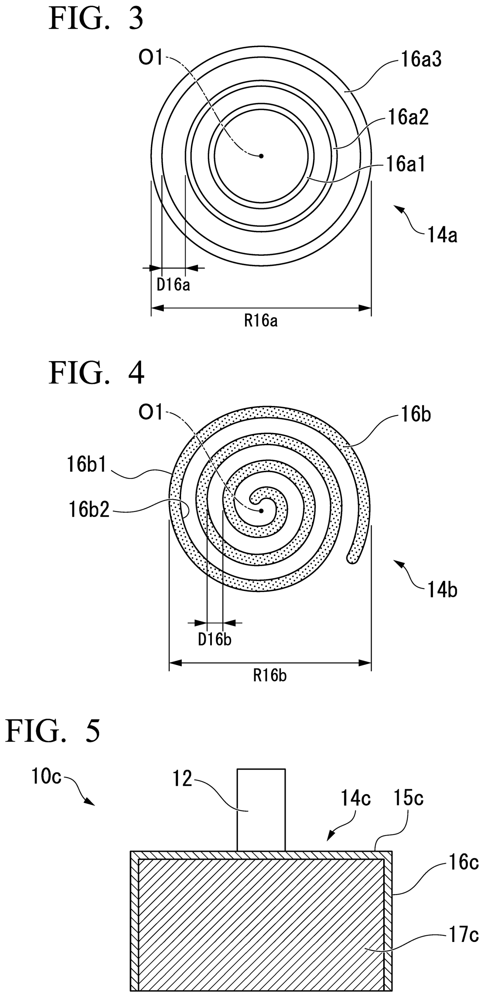

FIG. 3 is an end view of a lower end of the irradiation body 14a. The irradiation body 14a has a first wall portion 16a1, a second wall portion 16a2, and a third wall portion 16a3 in the order from the axis O1 toward the outside. In the bottom view, the first wall portion 16a1, the second wall portion 16a2, and the third wall portion 16a3 are positioned on concentric circles centered on the tube axis O1. That is, the irradiation body 14a has three coaxial wall portions in multiple layers. The first wall portion 16a1, the second wall portion 16a2, and the third wall portion 16a3 have a linear shape in a plan view as viewed from the second electrode 20 toward the first electrode 10. That is, the first wall portion 16a1, the second wall portion 16a2, and the third wall portion 16a3 are linear portions of the irradiation body 14a. Since the molecule introduction device has the wall portion in multiple layers, the introduction efficiency can be further increased by more uniformly irradiating the irradiation target with the plasma over a wide area.

The outer diameter R16a of the wall portion 16a3 positioned on the outermost side is the same as the outer diameter 16a.

The first wall portion 16a1, the second wall portion 16a2, and the third wall portion 16a3 are spaced apart from each other.

A distance D16a between the adjacent wall portions can be appropriately determined in consideration of a desired irradiation area, the outer diameter R16a, and the like, and is, for example, 1 to 5 mm.

Furthermore, the irradiation body 14a in FIG. 1 has triple wall portions, but the present invention is not limited thereto and the irradiation body 14a may have double or quadruple wall portions, or more. From the viewpoint of ease of manufacturing the irradiation body 14a, the wall portions are preferably double to quintuple wall portions.

The multiple wall portions may have the same shape or different shapes. For example, a combination of a polygonal tubular wall portion and a cylindrical wall portion may be used.

The molecule introduction device of the present invention may have the irradiation body 14b shown in FIG. 4.

The irradiation body 14b is a wound body and has a spiral wall portion 16b in the bottom view. The wall portion 16b has a linear shape in a plan view as viewed from the second electrode 20 toward the first electrode 10. That is, the first wall portion 16b is a linear portion of the irradiation body 14b. Since the molecule introduction device has the wall portion 16b which is a wound body, the introduction efficiency can be further increased by more uniformly irradiating the irradiation target with plasma over a wide area.

The adjacent wall portions 16b are spaced apart from each other. That is, one surface (outer surface) 16b1 and the other surface (inner surface) 16b2 of the wall portion 16b are spaced apart from each other.

A distance D16b between the adjacent wall portions 16b can be appropriately determined in consideration of a desired irradiation area, the outer diameter R16a, and the like, and is, for example, 1 to 5 mm.

In the above-described embodiment, the inside of the wall portion is hollow, but the present invention is not limited thereto. An insulating member may be positioned inside the wall portion.

FIG. 5 is a longitudinal sectional view of the first electrode 10c. The first electrode 10c has an electrode body 12 and an irradiation body 14c.

The irradiation body 14c has an insulating core portion 17c, a substrate portion 15c positioned on a top surface of the insulating core portion 17c, and a wall portion 16c positioned on an outer surface of the insulating core portion 17c. That is, the inside of the wall portion 16c is filled with the insulating core portion 17c. The wall portion 16c has a linear shape in a plan view as viewed from the second electrode 20 toward the first electrode 10. That is, the first wall portion 16c is a linear portion of the irradiation body 14c.

Examples of the insulating core portion 17c include a columnar or polygonal prismatic insulating member and a cylindrical or polygonal tubular insulating member. Examples of the insulating member include a molded body of a resin, a molded body of a metal oxide, a molded body of glass, and a molded body of ceramics. Examples of the molded body of the resin include a molded body of polystyrene, a polyolefin, or a polyester. Examples of the molded body of the metal oxide include a molded body of aluminum oxide (alumina), titanium oxide (titania), or magnesium oxide (magnesia).

The shape of the insulating core portion 17c is not particularly limited and can be appropriately determined according to a desired shape of the wall portion 16c.

Examples of the substrate portion 15c include a conductive thin film and a conductive flat plate. A material of the substrate portion 15c is the same as the material of the substrate portion 15.

Examples of the wall portion 16c include a conductive thin film and a conductive flat plate. A material of the wall portion 16c is the same as the material of the wall portion 16.

A method of manufacturing the irradiation body 14c is described below. First, the insulating core portion 17c having any shape is obtained. A conductive thin film is formed on a surface of the insulating core portion 17c except for one end surface of the insulating core portion 17c. By providing a conductive thin film on a surface excluding the one end surface, the thin film on the top surface of the insulating core portion 17c serves as the substrate portion 15c, the thin film on the peripheral surface of the insulating core portion 17c serves as the wall portion 16c, and the wall portion 16c is open at one end surface (an end surface facing the second electrode) of the insulating core portion 17c.

Examples of a method of forming the thin film include plating, vapor deposition, and sputtering.

A thickness of the thin film (that is, a thickness of the wall portion 16c) is, for example, preferably 1 to 10 µm.

In the molecule introduction device, by forming the wall portion 16c which is a thin film on a surface of the insulating core portion 17c, the edge of the lower end of the wall portion 16c is sharp, and thus the plasma can be more easily generated.

Furthermore, in a case where the irradiation body has two or more wall portions, all the spaces between the wall portions may be filled with the insulating core portions, or a part of the spaces between the wall portions may be filled with the insulating core portions. That is, in a case where the irradiation body has two or more wall portions, at least one wall portion may be positioned on an outer surface of the insulating core portion.

In the above-described embodiment, the wall portion is positioned on the outermost side. However, the present invention is not limited thereto, and a part or the whole of the outer surface of the wall portion may be covered with an insulation body.

In the above-described embodiment, the wall portion is continuous in the radial direction, but the present invention is not limited to this and the wall portion may have one or more slits extending in the axial direction.

In addition, the irradiation body may be a member in which a groove is formed on one surface of a conductive flat plate. By forming a groove on one surface of the flat plate, a wall portion is formed adjacent to the formed groove.

### - Second Embodiment -

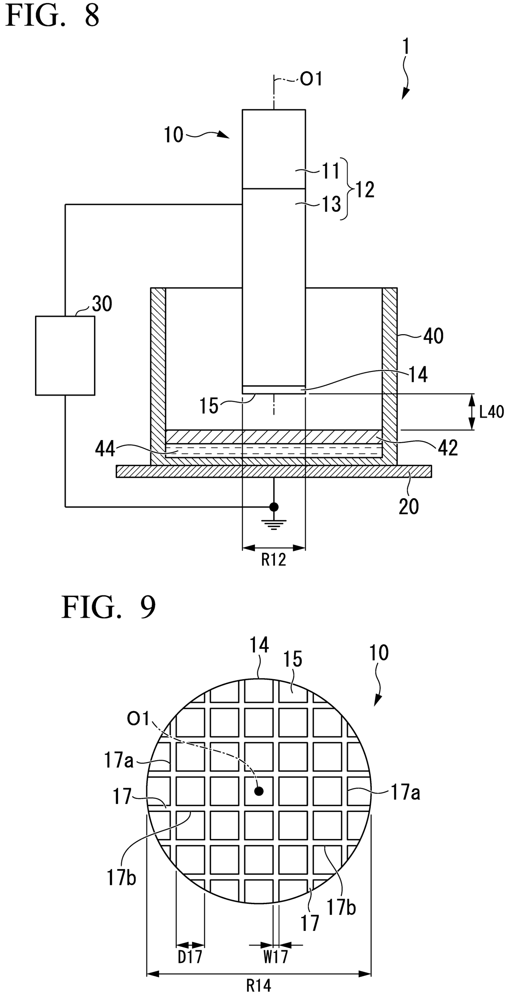

FIG. 8 is a cross-sectional view of a molecule introduction device according to a second embodiment of the present invention.

A molecule introduction device 1 of FIG. 8 has a first electrode 10, a second electrode 20, a power supply unit 30, and a container 40.

The second electrode 20 is spaced apart from the first electrode 10 and positioned below a lower end (distal end) of the first electrode 10. That is, the second electrode 20 is positioned beyond the distal end of the first electrode 10, and the first electrode 10 and the second electrode 20 face each other. In the present embodiment, the lower end surface of the first electrode 10 is an irradiation surface 15 facing the second electrode 20.

The power supply unit 30 is connected to the first electrode 10 and the second electrode 20.

The container 40 is positioned between the first electrode 10 and the second electrode 20. The container 40 is disposed to face the second electrode 20 and spaced apart from the first electrode 10.

The first electrode 10 has an electrode body 12 and an irradiation body 14 provided at a distal end (lower end) of the electrode body 12. Furthermore, in the present invention, the first electrode 10 may be composed of only the irradiation body 14.

In the present embodiment, the first electrode 10 is a high-voltage electrode.

The electrode body 12 has a dielectric 11 and a conductive coating film 13. In the present embodiment, the power supply unit 30 is connected to the conductive coating film 13.

The dielectric 11 is a rod-like body that extends in one direction.

The dielectric 11 may have a hollow structure such as a cylindrical shape and a polygonal tubular shape, or may also have a solid structure such as a columnar shape and a polygonal prismatic shape. In a case where the dielectric 11 has the solid structure among those, the durability is increased and the manufacture is facilitated.

Examples of the dielectric 11 include a molded body of a resin, a molded body of a metal oxide, a molded body of glass, and a molded body of ceramics.

The conductive coating film 13 covers a part of the outer surface of the dielectric 11 on the lower end side and opens the irradiation surface 15. The conductive coating film 13 may cover the entire outer surface of the dielectric 11 except for the irradiation surface 15.

Examples of the conductive coating film 13 include a foil or vapor deposition film of a metal such as stainless steel, copper, and tungsten, and a coating film of carbon.

A thickness of the conductive coating film 13 is, for example, 1 to 10 µm.

A thickness (outer diameter) R12 of the electrode body 12 is, for example, 3 to 50 mm. In a case where the electrode body 12 has the polygonal cylindrical shape or the polygonal prismatic shape, the outer diameter R12 is a diameter of the circumscribed circle of the transverse cross-section of the electrode body 12.

The irradiation body 14 is positioned at a lower end of the electrode body 12 and electrically connected to the conductive coating film 13.

As shown in FIG. 9, the first electrode 10 has a mesh-shaped irradiation body 14 from the bottom view. The irradiation body 14 has, as the linear portion, one or more first conductive wires 17a extending in one direction of a surface direction of the irradiation surface 15 and one or more second conductive wires 17b intersecting the first conductive wires 17a. These first conductive wires 17a and second conductive wires 17b are linear portions of the irradiation body 14. In the present embodiment, the six first conductive wires 17a and the six second conductive wires 17b intersect substantially perpendicularly, and the conductive wires form a mesh pattern. Furthermore, the number of conductive wires forming the mesh pattern is not limited to 6 × 6. The number of conductive wires forming the mesh pattern can be appropriately determined to be 1 × 1 (cross shape) or more. An upper limit of the number of conductive wires is not particularly limited, but examples thereof include 10 × 10. In addition, the number of first conductive wires 17a and the number of second conductive wires 17b may be the same as or different from each other.

Furthermore, examples of the irradiation body 14 include an irradiation body formed by combining linear conductive members, an irradiation body formed by etching a flat plate-shaped conductive member, and an irradiation body formed by pattern printing.

The outer edge shape of the irradiation body 14 in FIG. 9 is a substantially circular shape, but the outer edge shape of the irradiation body 14 may be an ellipse or a polygon.

Examples of a material of the irradiation body 14 include metals such as stainless steel, copper, and tungsten, and carbon.

The outer diameter R14 of the irradiation body 14 can be appropriately determined in consideration of the size of the container 40, and the like. The outer diameter R14 is, for example, preferably 3 to 50 mm, more preferably 5 to 40 mm, and still more preferably 10 to 30 mm. In a case where the outer diameter R14 is equal to or more than the lower limit value, the efficiency of introducing the molecules into the target cells and the like (which may hereinafter be simply referred to as an "introduction efficiency") can be further increased by performing plasma irradiation over a wider area. In a case where the outer diameter R14 is equal to or less than the upper limit value, plasma can be generated more stably.

In addition, the outer diameter R14 may be the same as or different from the outer diameter R12. From the viewpoint of increasing the introduction efficiency, it is preferable that the outer diameter R14 is equal to or more than the outer diameter R12.

In a case where the bottom view shape of the irradiation body 14 is a polygonal shape, the outer diameter R14 is a diameter of a circumscribed circle of the bottom view shape of the irradiation body 14.

The cross-sectional shape of the first conductive wire 17a (the shape of the cross-section of the first conductive wire 17a perpendicular to the longitudinal direction) may be a circle or a polygon. In a case where the cross-section of the first conductive wire 17a is circular, a shape thereof may be a true circle or an ellipse.

From the viewpoint of easily forming the first conductive wire 17a, the cross-sectional shape of the first conductive wire 17a is preferably a triangle or a quadrangle.

The cross-sectional shape of the second conductive wire 17b is the same as the cross-sectional shape of the first conductive wire 17a. The cross-sectional shape of the second conductive wire 17b may be the same as or different from the cross-sectional shape of the first conductive wire 17a.

The bottom view shape of the first conductive wire 17a may be a straight line, a curve, or a wavy line. In addition, the first conductive wire 17a may be a broken line.

The bottom view shape of the second conductive wire 17b is the same as the bottom view shape of the first conductive wire 17a. The bottom view shape of the second conductive wire 17b may be the same as or different from the bottom view shape of the first conductive wire 17a.

A width W17 of the first conductive wire 17a (the width of the linear portion in the plan view) is, for example, preferably 1 to 1,000 µm, more preferably 1 to 200 µm, and still more preferably 1 to 100 µm. In a case where the width W17 is equal to or more than the lower limit value, the strength of the first conductive wire 17a can be increased. In a case where the width W17 is equal to or less than the upper limit value, the electric field strength can be further increased.

The width of the second conductive wire 17b (the width of the linear portion in the plan view) is the same as the width W17 of the first conductive wire 17a. The width of the second conductive wire 17b may be the same as or different from the width W17 of the first conductive wire 17a as long as it is within the above range.

A height of the first conductive wire 17a (a distance from the proximal end to the distal end) is, for example, preferably 1 to 100 µm, and more preferably 5 to 10 µm. In a case where the height of the first conductive wire 17a is equal to or more than the lower limit value, the first conductive wire 17a is less likely to be peeled off from the base material. In a case where the height of the first conductive wire 17a is equal to or less than the upper limit value, the strength of the first conductive wire 17a can be increased.

The height of the second conductive wire 17b is the same as the height of the first conductive wire 17a. The height of the second conductive wire 17b may be the same as or different from the height of the first conductive wire 17a.

A separation distance D17 between the first conductive wires 17a can be appropriately determined in consideration of the outer diameter R14, and is, for example, preferably 0.1 to 10 mm, and more preferably 0.2 to 5 mm. In a case where the separation distance D17 is equal to or more than the lower limit value, plasma can be more stably generated. In a case where the separation distance D17 is equal to or less than the upper limit value, plasma can be more uniformly applied to the irradiation range.

The separation distance between the second conductive wires 17b is the same as the separation distance D17 between the first conductive wires 17a. The separation distance between the second conductive wires 17b may be the same as or different from the separation distance D17 between the first conductive wires 17a.

It should be noted that the length of the linear portion per cm² in the plan view is more than 20 mm, preferably 40 mm to 1,000 mm, and more preferably 80 mm to 400 mm. In a case where the width and the length are within the above ranges, the efficiency of introducing molecules can be further remarkably increased. In this context, the length is a total length of all of the first conductive wires and the second conductive wires.

In addition, in the plan view, it is preferable that the first conductive wire and the second conductive wire form at least one polygon, and it is more preferable that the first conductive wire and the second conductive wire form a plurality of polygons and the polygon is a triangle, a quadrangle, or a hexagon. In a case where the first conductive wire and the second conductive wire form a plurality of polygons, a structure of the irradiation body in the plan view is a triangular lattice structure (in a case where the polygon is a triangle), a quadrangular lattice structure (in a case where the polygon is a quadrangle), or a honeycomb structure (in a case where the polygon is a hexagon). In the plan view, the shape formed by the first conductive wire and the second conductive wire may be a shape other than a polygonal shape (an indefinite shape, a star shape, or the like) without departing from the present invention, but from the viewpoint of ease of manufacturing the irradiation body and uniform plasma irradiation, the shape is preferably the polygonal shape.

In the context of the present specification, the terms "first conductive wire" and "second conductive wire" are used for convenience, but this does not require that the irradiation body is composed of two or more wires. The irradiation body in the present embodiment may be configured such that the linear portions form two or more line segments having an intersection in the plan view.

In a case where the electrode body 12 has a hollow structure, the hollow portion may be open to the irradiation surface 15 or may be closed.

The second electrode 20 may function as a ground electrode, and examples thereof include a flat plate-shaped electrode.

Examples of a material of the second electrode 20 include metals such as stainless steel, copper, and tungsten, and carbon.

In the plan view, it is preferable that the area of the second electrode 20 is larger than the area of the first electrode 10. In a case where the area of the second electrode 20 is large, plasma can be irradiated from the first electrode 10 to the irradiation target over a wider area.

The power supply unit 30 may apply a voltage between the first electrode 10 and the second electrode to generate plasma between both electrodes. The power supply unit 30 switches the start or the stop of the supply of electricity to the electrodes. In addition, the power supply unit 30 adjusts a voltage and a frequency applied to the electrodes. Examples of the power supply unit 30 include a circuit including an inverter that is connected to an external power supply. In addition, examples of the power supply unit 30 include a secondary battery.

In the present embodiment, the container 40 functions as an irradiation field. The irradiation field may be any place where an irradiation target is placed in a method of introducing molecules to be described below. Therefore, in a case where the container 40 is not included in the configuration of the molecule introduction device 1, the second electrode 20 on which the container 40 is placed may be used as the irradiation field.

The container 40 may be any container as long as it can accommodate the irradiation target, and examples thereof include wells of a microplate.

The inner bottom surface of the container 40 is spaced apart from the distal end of the first electrode 10 (that is, the irradiation body 14).

The material of the container 40 may be a conductive material or an insulating material. From the viewpoint of preventing the occurrence of local discharge due to a high voltage, the insulating material is preferable. Examples of the insulating material include a resin, ceramics, and glass. Examples of the resin include polystyrene, a polyolefin, and a polyester.

### (Method of Using Molecule Introduction Device)

A method of using the molecule introduction device according to the second embodiment (that is, a method of introducing molecules) can be carried out in the same manner as the above-described method of using the molecule introduction device according to the first embodiment.

In the above-described embodiment, the electrode body has a dielectric and a conductive coating film, but the present invention is not limited thereto and the electrode body may consist of only a conductive material.

### Examples

### (Raw Materials Used)

· Target cells and the like: HEK293F (Thermo Fisher Scientific, R79007) cell.
· Aqueous liquid of molecule: DNA Solution (pCMC-eGFP).
· Culture medium: HE100 (manufactured by Gmep Co., Ltd.).
· Constituent substance: Penicillin and streptomycin.

### (Example 1)

A molecule introduction device similar to the molecule introduction device 1 shown in FIG. 1 was manufactured, except that the first electrode 10 was composed of only the irradiation body 14 under the following specifications.

### <Specifications>

· First electrode: An electrode in which a wall portion is formed by forming a metal on a surface of a columnar base material of aluminum oxide. A copper-made cylinder with a wall portion thickness of 5 µm and an outer diameter of 3 mm (that is, the linear portion in the plan view has a width of 5 µm and a length of 9.42 mm).
· Second electrode: A copper-made flat plate having a thickness of 3 mm.
· Container: A 6-well culture plate.

The inside of each well of the culture plate was coated with a scaffold material at 0.5 µg/cm². A culture medium, target cells and the like, and an antibiotic were placed in the coated wells, and the culture was performed at 37°C for 48 hours in an environment with a CO₂ concentration of 5% by volume (pre-culture). The number of cells after the pre-culture was 8.6 × 10⁵ cells/well.

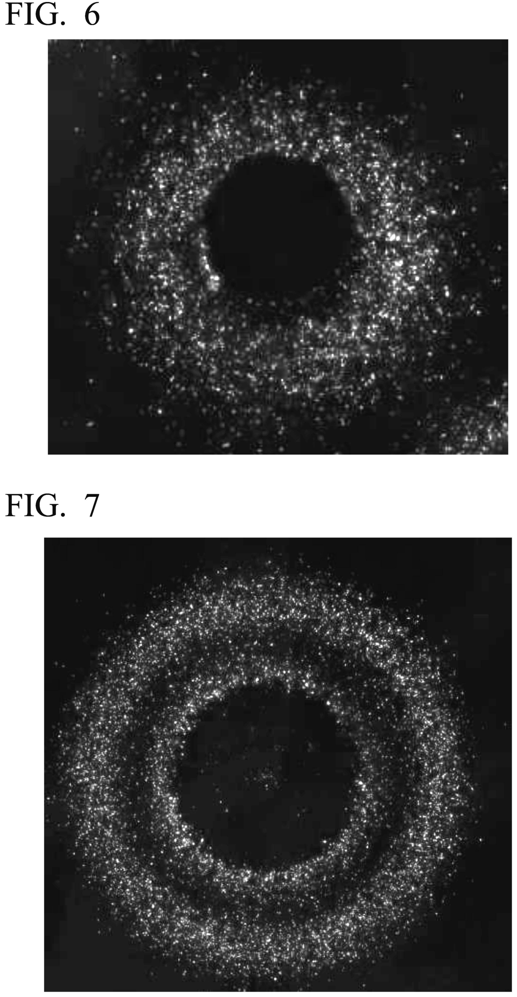

Next, the culture medium was removed and 57.5 µL of an aqueous liquid of molecules was added to the wells (contact step). The target cells and the like, and the irradiation target including the molecules in the wells were irradiated with plasma under the following irradiation conditions to introduce the molecules into the target cells and the like, thereby obtaining introduced cells and the like (irradiation step). The obtained introduced cells and the like were cultured in a culture medium at 37°C for 24 hours, and green fluorescence was observed with a fluorescence microscope (BZ-X800, manufactured by Keyence Corporation). The results are shown in FIG. 6.

### <Irradiation Conditions>

· Alternating voltage : 16 kVpp.
· Frequency of alternating current: 50 kHz.
· Plasma irradiation time: 40 msec.
· Distance from lower end of first electrode to irradiation target: 2 mm.

### (Example 2)

The introduced cells and the like were obtained in the same manner as in Example 1, except that the first electrode and the second electrode were changed according to the following specifications, and green fluorescence was observed. The results are shown in FIG. 7.

### <Specifications of Electrode>

· First electrode: An electrode composed of three hollow cylinders composed only of wall portions. A copper-made cylinder with a wall portion thickness of 0.5 mm and an outer diameter of 3 mm, 6 mm, or 10 mm (that is, the linear portion in the plan view has a width of 500 µm, and a length of 9.42 mm, 18.84 mm, and 31.4 mm).
· Second electrode: An aluminum-made flat plate having a thickness of 10 mm.

As shown in FIGs. 6 and 7, in any of Examples 1 and 2, green fluorescence indicating that DNA had been introduced was observed along the wall portion of the first electrode.

From these results, it was confirmed that by applying the present invention, the molecules were introduced over a wide area along the wall portion of the first electrode, and the efficiency of introducing molecules into the target cell was increased.

### (Example 3)

A molecule introduction device similar to the molecule introduction device 1 of FIG. 8 was manufactured under the following specifications.

### <Specifications>

· First electrode: The dielectric was a ceramic cylinder having an outer diameter of 10 mm. The conductive coating film was a copper plating having a thickness of 5 µm. The irradiation body had a 4 × 4 arrangement of conductive wires (width: 200 µm) placed at a separation distance of 2 mm within a circle with an outer diameter of 10 mm (a total length of conductive wires per cm² was 80 mm). The conductive wire was formed by plating.
· Second electrode: An aluminum-made flat plate having a thickness of 10 mm.
· Container: A 6-well culture plate.

The inside of each well of the culture plate was coated with a scaffold material at 0.5 µg/cm². A culture medium, target cells and the like, and an antibiotic were placed in the coated wells, and the culture was performed at 37°C for 48 hours in an environment with a CO₂ concentration of 5% by volume (pre-culture). The number of cells after the pre-culture was 8.6 × 10⁵ cells/well.

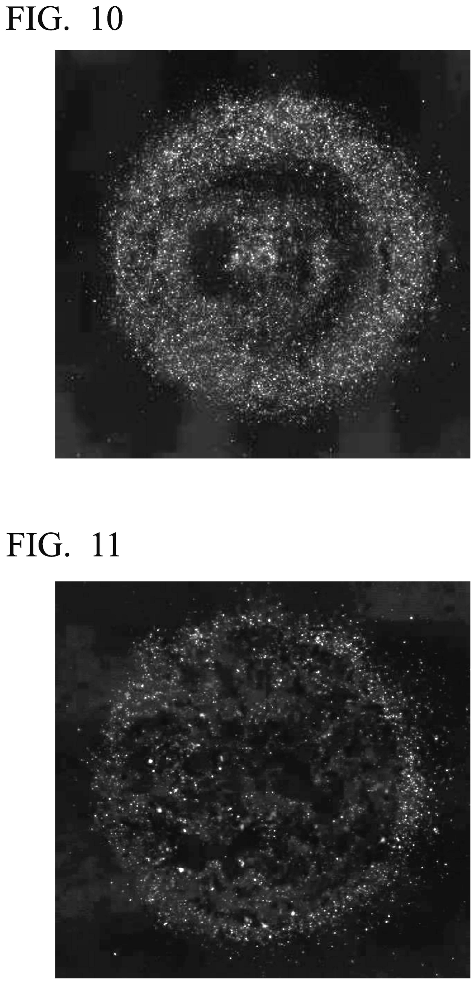

Next, the culture medium was removed and 57.5 µL of an aqueous liquid of molecules was added to the wells (contact step). The target cells and the like, and the irradiation target including the molecules in the wells were irradiated with plasma under the following irradiation conditions to introduce the molecules into the target cells and the like, thereby obtaining introduced cells and the like (irradiation step). The obtained introduced cells and the like were cultured in a culture medium at 37°C for 24 hours, and green fluorescence was observed with a fluorescence microscope (BZ-X800, manufactured by Keyence Corporation). The results are shown in FIG. 10.

### <Irradiation Conditions>

· Alternating voltage: 20 kVpp.
· Frequency of alternating current: 50 kHz.
· Plasma irradiation time: 40 msec.
· Distance from lower end of first electrode to irradiation target: 0.8 mm.

### (Example 4)

The introduced cells and the like were obtained in the same manner as in Example 3, except that the first electrode was set according to the following specifications and the irradiation conditions were set to the following conditions, and the green fluorescence was observed. The results are shown in FIG. 11.

### <Specifications of First Electrode>

· First electrode: The dielectric was a ceramic cylinder having an outer diameter of 10 mm. The conductive coating film was a copper plating having a thickness of 5 µm. The irradiation body had a 19 × 19 arrangement of conductive wires (width: 100 µm) placed at a separation distance of 0.5 mm within a circle with an outer diameter of 10 mm (a total length of conductive wires per cm² was 380 mm). The conductive wire was formed by plating.

### <Irradiation Conditions>

· Alternating voltage: 20 kVpp.
· Frequency of alternating current: 50 kHz.
· Plasma irradiation time: 20 msec
· Distance from lower end of first electrode to irradiation target: 0.8 mm.

As shown in FIGs. 10 and 11, in any of Examples 3 and 4, green fluorescence indicating that DNA had been introduced into the cells was observed in the entire lower portion of the electrode.

From these results, it was confirmed that the application of the present invention makes it possible to increase the efficiency of introducing molecules into target cells.

### REFERENCE SIGNS LIST

1 Molecule introduction device
10, 10c First electrode
11 Dielectric
12 Electrode body
13 Conductive coating film
14, 14a, 14b, 14c Irradiation body
15 Irradiation surface
16, 16a1, 16a2, 16a3, 16b, 16c Wall portion
17a First conductive wire
17b Second conductive wire
20 Second electrode
30 Power supply unit
40 Container
42 Molecule
44 Target cells and the like
50 Insulating coating film
O1 Tube axis
θ1 Angle

## Claims

1. A molecule introduction device comprising:
a first electrode;
a second electrode that is spaced apart from the first electrode and positioned in a direction in which a distal end of the first electrode faces;
an irradiation field that is positioned between the first electrode and the second electrode, and positioned to be spaced apart from the distal end of the first electrode; and
a power supply unit configured to apply a voltage between the first electrode and the second electrode,
wherein the first electrode has an irradiation body at a distal end on a second electrode side,
the irradiation body has conductivity and has a linear portion having a linear shape in a plan view as viewed from the second electrode toward the first electrode, and
in the plan view, the linear portion has a width of 1 µm to 1,000 µm and a length of more than 3.14 mm.

2. The molecule introduction device according to Claim 1,
wherein the irradiation body has, as the linear portion, one or more linear wall portions in the plan view, and
the wall portion has a cylindrical or polygonal tubular shape with its tube axis aligned with a direction from the first electrode toward the second electrode.

3. The molecule introduction device according to Claim 2,
wherein an outer diameter of the wall portion is more than 1 mm.

4. The molecule introduction device according to Claim 2 or 3,
wherein the irradiation body has two or more of the wall portions, and
the two or more wall portions form a multiple-wall structure and are spaced apart from each other.

5. The molecule introduction device according to Claim 2 or 3,
wherein the wall portion is a wound body with its tube axis aligned with the direction from the first electrode toward the second electrode, and
adjacent wall portions are spaced apart from each other.

6. The molecule introduction device according to Claim 2 or 3,
wherein the irradiation body has an insulating core portion, and
at least one of the wall portions is positioned on an outer surface of the insulating core portion.

7. The molecule introduction device according to Claim 1,
wherein the irradiation body has, as the linear portion, one or more first conductive wires extending in one surface direction in the plan view and one or more second conductive wires intersecting the first conductive wires.

8. The molecule introduction device according to Claim 7,
wherein the first electrode has an electrode body,
the electrode body has a dielectric that extends in a direction from the first electrode toward the second electrode, and a conductive coating film configured to cover a part or an entirety of an outer surface of the dielectric,
the conductive coating film is connected to the power supply unit, and
the first conductive wire and the second conductive wire are electrically connected to the conductive coating film.

9. The molecule introduction device according to Claim 7 or 8,
wherein a width of the first conductive wire is 1 to 1,000 µm, and
a width of the second conductive wire is 1 to 1,000 µm.

10. The molecule introduction device according to Claim 7 or 8,
wherein two or more of the first conductive wires are provided, with a separation distance between the first conductive wires of 0.1 to 10 mm, and
two or more of the second conductive wires are provided, with a separation distance between the second conductive wires of 0.1 to 10 mm.

11. The molecule introduction device according to Claim 7 or 8,
wherein in the plan view, the first conductive wire and the second conductive wire form at least one polygon.

12. The molecule introduction device according to Claim 7 or 8,
wherein in the plan view, the first conductive wire and the second conductive wire form a plurality of polygons, and
the polygon is a triangle, a quadrangle, or a hexagon.

13. The molecule introduction device according to Claim 1 or 2,
wherein in the plan view, the width of the linear portion is 1 µm to 1,000 µm, and
the length of the linear portion per cm² is 5 mm to 320 mm.

14. The molecule introduction device according to Claim 7 or 8,
wherein in the plan view, the width of the linear portion is 1 µm to 1,000 µm, and
a total length of the first conductive wire and the second conductive wire per cm² is 20 mm to 1,000 mm.
